(19) Europäisches Patentamt
European Patent Office
Office européen des brevets

(11) Veröffentlichungsnummer : **0 405 486 B1**

(12) # EUROPÄISCHE PATENTSCHRIFT

(45) Veröffentlichungstag der Patentschrift :
**22.07.92 Patentblatt 92/30**

(51) Int. Cl.$^5$ : **B01J 29/06,** C07C 45/38,
C07C 45/39

(21) Anmeldenummer : **90112226.7**

(22) Anmeldetag : **27.06.90**

(54) **Katalysator für selektive Oxidationsreaktionen.**

(30) Priorität : **30.06.89 DE 3921450**

(43) Veröffentlichungstag der Anmeldung :
**02.01.91 Patentblatt 91/01**

(45) Bekanntmachung des Hinweises auf die Patenterteilung :
**22.07.92 Patentblatt 92/30**

(84) Benannte Vertragsstaaten :
**BE DE ES FR GB IT NL**

(56) Entgegenhaltungen :
**EP-A- 0 106 674**
**EP-A- 0 275 892**
**DE-A- 3 037 536**
**US-A- 3 835 032**

(73) Patentinhaber : **HOECHST AKTIENGESELLSCHAFT**
**Postfach 80 03 20**
**W-6230 Frankfurt am Main 80 (DE)**

(72) Erfinder : **Beck, Horst-Philipp, Prof. Dr.**
**Scheidter Strasse 158**
**W-6600 Saarbrücken (DE)**
Erfinder : **Emig, Gerhard, Prof. Dr.**
**Vogelherd 157**
**W-8520 Erlangen (DE)**
Erfinder : **Wiesgickl, Günther, Dr.**
**Schillerring 23**
**W-8751 Grosswallstadt (DE)**
Erfinder : **Burg, Karlheinz, Dr.**
**Eichenweg 18**
**W-6200 Wiesbaden (DE)**
Erfinder : **Mück, Karl-Friedrich, Dr.**
**Schnitterweg 7**
**W-6200 Wiesbaden (DE)**

EP 0 405 486 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Silikatkatalysatoren, die für selektive Oxidationsreaktionen organischer Substanzen eingesetzt werden können, und ein Verfahren zur Herstellung dieser Katalysatoren.

Gerüstsilikatkatalysatoren, z.B. Zeolithe, werden großtechnisch bereits für die nichtoxidative Umwandlung von Kohlenwasserstoffen als Katalysatoren eingesetzt, z. B. beim katalytischen Cracken.

Es ist auch bekannt, daß organische Stoffe mit Hilfe von Zeolithkatalysatoren selektiv oxidiert werden können, wie dies z.B. bei der Acetonherstellung aus Propen der Fall ist. Hier wird bei einem Umsatz von 50 % eine Selektivität von 90 % erzielt. Aussagen über die Langzeitaktivität und das Alterungsverhalten werden dabei nicht gemacht.

Weiterhin wird angenommen, daß Zeolithe infolge ihrer besonderen Struktur zur Beschleunigung der Totaloxidation von organischen Stoffen zu $CO_2$ und Wasser neigen.

Die Herstellung von bestimmten Katalysatoren auf Zeolith-Basis wird in der DDR-Patentschrift 113 173 beschrieben. Hierbei erfolgt der Einbau bestimmter Metalle wie Vanadium und/oder Titan in die Zeolithe durch Ionenaustausch.

Für die industriell besonders bedeutsamen Oxidehydrierungen von $C_1$-$C_4$-Alkoholen, z.B. von Methanol zu Formaldehyd wird häufig ein Katalysator "Silber auf Bimstein" bzw. "Silber auf $Al_2O_3$" verwendet.
In der deutschen Patentschrift 30 37 536 wird auf die Herstellung eines Silberkatalysators eingegangen, der durch Tränken des Trägers, welcher aus $Al_2O_3$ und $SiO_2$ in Form von Cristobalit besteht, mit Silbernitratlösung synthetisiert wird.

Für die Herstellung von Formaldehyd wird auch ein Katalaysator beschrieben, bestehend aus einem Träger aus Metall oder Keramik, der mit metallischem Kupfer, Silber, Gold oder Eisen beschichtet oder imprägniert ist (DE-OS 28 16 471).

Die genannten Katalysatoren, die bei Verfahren zur Herstellung von Carbonylverbindungen durch oxidative Dehydrierung von $C_1$-$C_4$-Alkoholen eingesetzt werden, weisen jedoch nur relativ niedrige Ausbeuten an Endprodukt, bezogen auf den durch- und umgesetzten Rohstoff auf, weiterhin ist der Grad der Umwandlung des Rohstoffs und die Qualität der Produkte meist noch unbefriedigend. Als unerwünschtes Nebenprodukt wird bei den bekannten Verfahren häufig auch Ameisensäure gebildet.

Es sind auch Zeolithkatalysatoren beschrieben worden, die mit Silber und Salzen seltener Erden imprägniert und anschließend calciniert werden (US-A 3,835,032). Verwendung finden diese Katalysatoren bei der catalytischen Crackung von Kohlenwasserstoffen.

Es bestand daher die Aufgabe, andere Katalysatorsubstanzen zu finden, durch die das Verfahren zur Herstellung von Oxoverbindungen weiter verbessert wird.

Die Erfindung betrifft daher einen komplexen Silikatkatalysator mit der Formel

$$M^{n+}_{2/n}0 \cdot v\,Ag^0 \cdot w\,AgX \cdot Al_2O_3 \cdot x\,SiO_2 \cdot y\,H_2O \quad (1)$$

in der M ein Metallatom der Wertigkeit n, $Ag^0$ elementares Silber, X ein Halogenatom und v, w, x und y stöchiometrische Koeffizienten bedeuten. Als Metalle M finden Elemente der ersten und zweiten Hauptgruppe des periodischen Systems, vorzugsweise Natrium, Kalium, Calzium und Barium Verwendung. Anstelle der Metalle $M^{n+}$ können im Silikat auch die $H^+$- oder $NH_4^+$-Formen vorliegen. Bei hohen Reaktionstemperaturen wird die $NH_4^+$-Form in die $H^+$-Form überführt. Als Halogenatom X wird Brom, vorzugsweise aber Chlor eingesetzt. Die Koeffizienten v, w, x und y stellen Zahlen von 0,1 bis 7,5, vorzugsweise bis 4,6 dar. Ein besonders bevorzugter Silikat-Komplex besitzt die Formel

$$Na_2O \cdot 1{,}4\,Ag \cdot 0{,}4\,AgCl \cdot Al_2O_3 \cdot 2{,}47\,SiO_2 \cdot 3{,}9\,H_2O \quad (2)$$

Die Erfindung betrifft auch ein Verfahren zur Herstellung des komplexen Silikatkatalysators, bei dem (a) ein Gerüstsilikat mit einem Silbersalz umgesetzt wird, (b) das Silberionen enthaltende Gerüstsilikat mit Hilfe von Reduktionsmitteln in einen elementares Silber enthaltenden Gerüstsilikat-Komplex überführt wird, (c) dieser Komplex durch teilweise Reoxidation mit oxidierenden Mitteln in einen Silber(I)-ionen und elementares Silber enthaltenden Silikat-Komplex umgewandelt wird und (d) anschließend durch Behandlung mit einer Halogenidionen enthaltenden Lösung im alkalischen Medium zu einem Silikat-Komplex der Formel (1) umgewandelt wird.

Als Gerüstsilikate für die Herstellung der erfindungsgemäßen Katalysatoren können allgemein gängige Zeolithe verwendet werden.
Besonders geeignete Beispiele für derartige Silikate sind kommerziell vertriebene Produkte wie 6 $Na_2O \cdot 6\,Al_2O_3 \cdot 12\,SiO_2 \cdot 12\,H_2O$, bekannt als Molekularsieb ZA (Hersteller: Union Carbide, USA), sowie $Na_{1,8}H_{0,2}O \cdot Al_2O_3 \cdot 4{,}65\,SiO_2 \cdot 10{,}4\,H_2O$, bekannt als Baylith CP 190 (Hersteller: Bayer AG, Leverkusen, Bundesrepublik Deutschland). Der Wassergehalt der Zeolithe unterliegt starken Schwankungen. Vorzugsweise wird $Na_2O \cdot Al_2O_3 \cdot 2{,}47\,SiO_2 \cdot 3{,}9\,H_2O$, bekannt als Molekularsieb Z13X (Hersteller: Union Carbide, Corp., New York, USA) eingesetzt.

Zur Dotierung, d.h. zur Einlagerung in die Gerüstsilikate werden Lösungen eingesetzt, die Silberkationen enthalten. Die molare Silberionenkonzentration beträgt dabei 0,001 bis 10, vorzugsweise 0,01 bis 1 Mol/l. Die Umsetzung wird bei Raumtemperatur, d.h. bei 20-30 °C unter Rühren und Lichtausschluß durchgeführt. Die Behandlung des Gerüstsilikats mit der Silberionenlösung kann so oft durchgeführt werden, bis der gewünschte Austauschgrad gegenüber den Metallionen des Gerüstsilikats eingestellt worden ist. Zur weiteren Umsetzung wird das dotierte Silikat getrocknet. Eine ähnliche Beladung kann auch z.B im Imprägnierverfahren erreicht werden.

Die im Gerüstsilikat enthaltenen Silberionen werden anschließend mit Reduktionsmitteln in elementares Silber überführt. Diese Reduktion wird z.B. in üblicher Weise mit Wasserstoff durchgeführt bei Temperaturen zwischen 100 und 500 °C, vorzugsweise zwischen 300 und 400 °C. Der Partialdruck des Reduktionsmittels beträgt dabei $5 \bullet 10^3$ bis $5 \bullet 10^5$ Pa (0,05 bis 5,0 bar), vorzugsweise $4 \bullet 10^4$ bis $7{,}5 \bullet 10^4$Pa (0,4 bis 0,75 bar), wobei der absinkende Partialdruck ein Maß für die Reduktion ist.

Die teilweise Reoxidation der Stufe (c) wird durch Oxidationsmittel, z.B. Sauerstoff oder Halogen wie Brom oder Chlor durchgeführt. Hierbei wird unter Abspaltung von Wasser bzw. Halogenwasserstoff das elementare Silber teilweise in Silber(I)-Ionen bzw. Silberhalogenid umgewandelt. Die Reaktion findet bei Temperaturen zwischen 100 und 350 °C, vorzugsweise 250 bis 290 °C, und bei Halogen vorzugsweise zwischen 100 und 200 °C, bei einemPartialdruck von $5 \bullet 10^4$ bis $5 \bullet 10^5$Pa (0,05 bis 5,0 bar), vorzugsweise $4 \bullet 10^4$ bis $7{,}5 \bullet 10^4$Pa (0,4 bis 0,75 bar) statt. Die Behandlungsdauer bei der Oxidation reicht von 5 bis 60, vorzugsweise 10 bis 30 Minuten.

In der Stufe (d) wird der erhaltene Silikat-Komplex mit einer Halogenidionen enthaltenen, vorzugsweise chloridhaltigen wässrigen Lösung behandelt. Die molare Konzentration dieser Lösung beträgt 0,001 bis 2, vorzugsweise 0,01 bis 0,5. Vorzugsweise werden hierzu Halogenwasserstoffsäuren wie Salzsäure oder Bromwasserstoffsäure eingesetzt. Die Behandlung wird in einem Zeitraum von 2 bis 15, vorzugsweise 5 bis 10 Minuten durchgeführt. Anschließend wird ein Silberionenkomplexbildner, z.B. Ammoniak in 0,1 bis 0,3 molarer Lösung zugegeben. Gleichzeitig wird ein das Metallkation M in Formel (1) enthaltendes Salz, z. B. $NaNO_3$ im Überschuß zugegeben. Die Suspension wird anschließend für 20 bis 50 Minuten gerührt. Die Digerierung mit dem Komplexbildner und dem Salz kann mehrfach wiederholt werden.

Wird die Oxidation mit Halogen vorgenommen, erübrigt sich die Behandlung mit einer halogenidionenhaltigen Lösung. Die Nachbehandlung in Stufe (d) erfolgt dann nur mit einer alkalischen Lösung eines Komplexbildners in Gegenwart des Metallsalzes.

Der erhaltene Katalysator wird nach dem Trocknen zu Pellets von 1 bis 5 mm Größe verarbeitet und ist dann einsatzfähig.

Durch die Fällung des teilweise ionisch vorliegenden Silbers als Halogenid und dessen partielle Auswaschung mittels Komplexbildner verbleibt eine überraschenderweise aktiv und selektiv wirkende elementare Silberkomponente in der Silikatmatrix. Der so hergestellte Katalysator zeichnet sich durch hohe Langzeitaktivität, eine für die Umsetzung niedrigere notwendige Reaktionstemperatur im Vergleich zu Katalysatoren aus reinem Silber sowie durch höhere Umsätze und Selektivitäten bei der oxidativen Dehydrierung von Alkanolen, beispielsweise Methanol zu Formaldehyd im Vergleich zu reinem Silber bzw. Silber auf Trägern aus.

Der erfindungsgemäße Katalysator findet bei Oxidehydrierungsreaktionen Verwendung. Besonders eignet er sich zur Oxidehydrierung von Alkanolen mit 1 bis 4 C-Atomen im Alkylrest und insbesondere bei der oxidativen Dehydrierung von Methanol zu Formaldehyd wie in der deutschen Patentanmeldung P 39 21 452.4, Titel: "Verfahren Zur Herstellung von Carbonylverbindungen" beschrieben, die am selben Tag eingesicht worden ist.

## Beispiele

1) 10,71 g Zeolith 13X (Formel: $Na_2O \cdot Al_2O_3 \cdot 2{,}47\ SiO_2 \cdot 3{,}9\ H_2O$) wurden in ein Becherglas gegeben, mit 500 ml 0,1 n $AgNO_3$-Lösung versetzt und 4 Stunden unter Lichtausschluß kräftig gerührt. Nach Filtration wurde der Rückstand mit 50 ml Wasser gewaschen und erneut mit 400 ml 0,1 n Silbernitratlösung versetzt, 4 Stunden unter Lichtausschluß gerührt, anschließend filtriert und die erhaltene feste Substanz an der Luft getrocknet. 7,88 g dieser Substanz wurden bei 350 °C unter vermindertem Druck entwässert und dann mit Wasserstoff bei einem Druck von $6 \bullet 10^4$Pa (0,6 bar) für 20 Minuten bei der gleichen Temperatur reduktiv behandelt. Anschließend wurde der Wasserstoff entfernt und der Rückstand auf 270 °C abgekühlt. Die Festsubstanz wurde anschließend in Sauerstoff $6{,}7 \bullet 10^4$Pa (0,67 bar) für 8 Minuten bei dieser Temperatur behandelt und nach Entfernung des Sauerstoffs auf ca. 25 °C abgekühlt. 3,5 g dieser erhaltenen Substanz wurden in 200 ml Wasser aufgeschlämmt und unter Rühren 5 ml konzentrierte Salzsäure zugegeben. Fünf Minuten später erfolgte die Zudosierung von 25 ml halbkonzentrierter Ammoniaklösung (7 mol/l) und 17 g Natriumnitrat. Diese Suspension wurde 1 Stunde gerührt, anschließend filtriert und der Rückstand mit

insgesamt 200 ml Wasser gewaschen. Die erhaltene Substanz wurde an der Luft getrocknet und zu Pellets mit 6 mm Durchmesser gepresst, die anschließend zerkleinert wurden.

2) 8,51 g Zeolith 13X (Formel: $Na_2O{\cdot}Al_2O_3{\cdot}2,47\ SiO_2{\cdot}3,9\ H_2O$) wurden in ein Becherglas gegeben, mit 400 ml 0,1 n $AgNO_3$-Lösung versetzt und 3 Stunden unter Lichtausschluß kräftig gerührt. Nach Filtration wurde der Rückstand mit 50 ml Wasser gewaschen und erneut mit 400 ml 0,1 n Silbernitratlösung versetzt, 3 Stunden unter Lichtausschluß gerührt, anschließend filtriert und die erhaltene Feststubstanz an der Luft getrocknet. 6,35 g dieser Substanz wurden bei 330 °C unter vermindertem Druck entwässert und dann mit Wässerstoff bei einem Druck von $6,7{\bullet}10^4$Pa (0,67 bar) für 25 Minuten reduziert. Anschließend wurde die Substanz unter vermindertem Druck auf 170 °C abgekühlt, mit Chlor bei einem Druck von $6{\bullet}10^4$Pa (0,6 bar) für 30 Minuten oxidiert, und unter vermindertem Druck auf Raumtemperatur abgekühlt. 4 g der erhaltenen Substanz wurden in 200 ml Wasser aufgeschlämmt und 20 g Natriumnitrat sowie 30 ml halbkonzentrieite Ammoniaklösung zudosiert. Diese Suspension wurde 20 Minuten gerührt, dann filtriert und der Rückstand mit insgesamt 250 ml Wasser gewaschen. Die erhaltene Substanz wurde an der Luft getrocknet und zu Pellets mit 6 mm Durchmesser gepreßt, die anschließend zerkleinert wurden.

## Patentansprüche

### Patentansprüche für folgenden Vertragsstaaten: BE, DE, IT, NL, FR, GB

1. Komplexer Silikat-Katalysator der Formel

$$M^{n+}_{2/n}O \cdot v\ Ag^{O} \cdot w\ AgX \cdot Al_2O_3 \cdot x\ SiO_2 \cdot y\ H_2O \quad (1)$$

wobei M ein Metallatom der Wertigkeit n, $Ag^O$ elementares Silber, X ein Halogenatom und v, w, x und y Zahlen im Bereich von 0,1 bis 7,5 bedeuten.

2. Katalysator nach Anspruch 1, dadurch gekennzeichnet, daß er der Formel

$$Na_2O \cdot 1,4\ Ag \cdot 0,4\ AgCl \cdot Al_2O_3 \cdot 2,47\ SiO_2 \cdot 3,9\ H_2O \quad (2).$$

entspricht.

3. Verfahren zur Herstellung eines komplexen Silikat-Katalysators, dadurch gekennzeichnet, daß (a) ein Gerüstsilikat mit einem Silbersalz umgesetzt wird, (b) das Silberionen enthaltende Gerüstsilikat mit Hilfe von Reduktionsmitteln in einen elementares Silber enthaltenden Silikat-Komplex überführt wird, (c) dieser Komplex durch teilweise Reoxidation mit oxidierenden Mitteln in einen Silber(I)-ionen und elementares Silber enthaltenden Silikat-Komplex umgewandelt wird und (d) anschließend durch Behandlung mit einer Halogenidionen enthaltenden Lösung im alkalischen Medium zu einem Silikat-Komplex-gemäß Anspruch 1 umgewandelt wird.

4. Verfahren nach Anspruch 4, dadurch gekennzeichnet, daß als Reduktionsmittel Wasserstoff verwendet wird.

5. Verfahren nach Anspruch 3 oder 4, dadurch gekennzeichnet, daß als oxidierendes Mittel Sauerstoff oder Halogen, vorzugsweise Chlor, eingesetzt und bei der Reoxidation Wasser oder Halogenwasserstoff freigesetzt wird.

6. Verfahren nach einem oder mehreren der Ansprüche 3 bis 5, dadurch gekennzeichnet, daß bei der Reoxidation mit Halogen in Stufe (c) die sich anschließende Behandlung (d) nur mit einer alkalischen Lösung eines Komplexbildners in Gegenwart eines das Metallion M der Formel (1) enthaltendes Salz durchgeführt wird.

7. Verfahren nach einem oder mehreren der Ansprüche 3 bis 6, dadurch gekennzeichnet, daß die im Komplex in Stufe (c) gebildeten Silber(I)-ionen in Form von Silberchlorid vorliegen.

8. Verfahren nach einem oder mehreren der Ansprüche 3 bis 7, dadurch gekennzeichnet, daß das Gerüstsilikat in Stufe (a) mit einer $Ag^+$-ionen enthaltenden Lösung in einer Konzentration von 0,001 bis 10 Mol/l, vorzugsweise 0,01 bis 1 Mol/l, bei Raumtemperatur unter Lichtausschluß behandelt wird.

9. Verfahren nach einem oder mehreren der Ansprüche 3 bis 8, dadurch gekennzeichnet, daß die Reduktion in Stufe (b) bei 100 bis 500 °C, vorzugsweise bei 300 bis 400 °C bei einem Partialdruck des Reduktionsmittels von 0,05 bis 5,0 bar, vorzugsweise 0,4 bis 0,75 bar, durchgeführt wird.

10. Verfahren nach einem oder mehreren der Ansprüche 3 bis 9, dadurch gekennzeichnet, daß die Reoxidation in Stufe (c) bei 100 bis 350 °C, vorzugsweise 250 bis 290 °C einem Partialdruck des Oxidationsmittels von 0,05 bis 5,0 bar, vorzugsweise 0,4 bis 0,75 bar, während 5 bis 60, vorzugsweise 10 bis 30 Minuten durchgeführt wird.

11. Verfahren nach Anspruch 10, dadurch gekennzeichnet, daß die Reoxidation mit Halogen als Oxidationsmittel vorzugsweise bei 100-200 °C durchgeführt wird.

12. Verfahren nach einem oder mehreren der Ansprüche 3 bis 11, dadurch gekennzeichnet, daß die Halogenidionen enthaltende Lösung in Stufe (d) eine molare Konzentration von 0,001 bis 2, vorzugsweise 0,01 bis 0,5 aufweist.

**Patentanprüche für folgenden Vertragsstaat: ES**

1. Verfahren zur Herstellung eines komplexen Silikat-Katalysators, der Formel

$$M^{n+}_{2/n}O \cdot v\ Ag^{O} \cdot w\ AgX \cdot Al_2O_3 \cdot x\ SiO_2 \cdot y\ H_2O \quad (1)$$

wobei M ein Metallatom der Wertigkeit n, $Ag^{O}$ elementares Silber, X ein Halogenatom und v, w, x und y Zahlen im Bereich von 0,1 bzw. 7,5 bedeuten, dadurch gekennzeichnet, daß (a) ein Gerüstsilikat mit einem Silbersalz umgesetzt wird, (b) das Silberionen enthaltende Gerüstsilikat mit Hilfe von Reduktionsmitteln in einen elementares Silber enthaltenden Silikat-Komplex überführt wird, (c) dieser Komplex durch teilweise Reoxidation mit oxidierenden Mitteln in einen Silber(I)-ionen und elementaies Silber enthaltenden Silikat-Komplex umgewandelt wird und (d) anschließend durch Behandlung mit einer Halogenidionen enthaltenden Lösung im alkalischen Medium zu einem Silikat-Komplex der Formel (1) umgewandelt wird.

2. Verfahren nach Anspruch 1, dadurch gekennzeichnet, daß der Katalysator der Formel

$$Na_2O \cdot 1{,}4\ Ag \cdot 0{,}4\ AgCl \cdot Al_2O_3 \cdot 2{,}47\ SiO_2 \cdot 3{,}9\ H_2O \quad (2).$$

entspricht.

3. Verfahren nach Anspruch 1 oder 2, dadurch gekennzeichnet, daß als Reduktionsmittel Wasserstoff verwendet wird.

4. Verfahren nach einem oder mehreren der Ansprüche 1 bis 3, dadurch gekennzeichnet, daß als.oxidierendes Mittel Sauerstoff oder Halogen, vorzugsweise Chlor, eingesetzt und bei der Reoxidation Wasser oder Halogenwasserstoff freigesetzt wird.

5. Verfahren nach einem oder mehreren der Ansprüche 1 bis 4, dadurch gekennzeichnet, daß bei der Reoxidation mit Halogen in Stufe (c) die sich anschließende Behandlung (d) nur mit einer alkalischen Lösung eines Komplexbildners in Gegenwart eines das Metallion M der Formel (1) enthaltendes Salz durchgeführt wird.

6. Verfahren nach einem oder mehreren der Ansprüche 1 bis 5, dadurch gekennzeichnet, daß die im Komplex in Stufe (c) gebildeten Silber(I)-ionen in Form von Silberchlorid vorliegen.

7. Verfahren nach einem oder mehreren der Ansprüche 1 bis 6, dadurch gekennzeichnet, daß das Gerüstsilikat in Stufe (a) mit einer $Ag^{+}$-ionen enthaltenden Lösung in einer Konzentration von 0,001 bis 10 Mol/l, vorzugsweise 0,01 bis 1 Mol/l, bei Raumtemperatur unter Lichtausschluß behandelt wird.

8. Verfahren nach einem oder mehreren der Ansprüche 1 bis 7, dadurch gekennzeichnet, daß die Reduktion in Stufe (b) bei 100 bis 500 °C, vorzugsweise bei 300 bis 400 °C bei einem Partialdruck des Reduktionsmittels von 0,05 bis 5,0 bar, vorzugsweise 0,4 bis 0,75 bar, durchgeführt wird.

9. Verfahren nach einem oder mehreren der Ansprüche 1 bis 8, dadurch gekennzeichnet, daß die Reoxidation in Stufe (c) bei 100 bis 350 °C, vorzugsweise 250 bis 290 °C, einem Partialdruck des Oxidationsmittels von 0,05 bis 5,0 bar, vorzugsweise 0,4 bis 0,75 bar, während 5 bis 60, vorzugsweise 10 bis 30 Minuten durchgeführt wird.

10. Verfahren nach Anspruch 9, dadurch gekennzeichnet, daß die Reoxidation mit Halogen als Oxidationsmittel vorzugsweise bei 100-200 °C durchgeführt wird.

11. Verfahren nach einem oder mehreren der Ansprüche 1 bis 10, dadurch gekennzeichnet, daß die Halogenidionen enthaltende Lösung in Stufe (d) eine molare Konzentration von 0,001 bis 2, vorzugsweise 0,01 bis 0,5 aufweist.

## Claims

**Claims for the following Contracting States: BE, DE, IT, NL, FR, GB**

1. A complex silicate catalyst of the formula

$$M^{n+}_{2/n}O \cdot v\ Ag^{O} \cdot w\ AgX \cdot Al_2O_3 \cdot x\ SiO_2 \cdot y\ H_2O \quad (1)$$

in which M denotes a metal atom of valence n, $Ag^{O}$ denotes elementary silver, X denotes a halogen atom and v, w, x and y denote numbers in the range from 0.1 to 7.5

2. The catalyst as claimed in claim 1, which corres ponds to the formula

$$Na_2O\ .\ 1.4\ Ag \cdot 0.4\ AgCl \cdot Al_2O_3 \cdot 2.47\ SiO_2 \cdot 3.9\ H_2O \quad (2).$$

3. A process for the preparation of a complex silicate catalyst, which comprises (a) reacting a skeleton silicate with a silver salt, (b) converting the skeleton silicate containing silver ions by means of reducing agents into a silicate complex containing elementary silver, (c) converting this complex by partial re-oxidation with

oxidizing agents into a silicate complex containing silver(I) ions and elementary silver and (d) then converting the complex into a silicate complex of the formula (1) by treatment with a solution containing halide ions in an alkaline medium.

4. The process as claimed in claim 3, wherein the reducing agent used is hydrogen.

5. The process as claimed in claim 3 or 4, wherein the oxidizing agent employed is oxygen or halogen, pre ferably chlorine, and water or hydrogen halide is liberated in the course of the re-oxidation.

6. The process as claimed in one or more of claims 3 to 5, wherein, when the re-oxidation is carried out with a halogen in stage (c), the subsequent treatment (d) is only carried out with an alkaline solution of a complex-former in the presence of a salt containing the metal ion M of formula (1).

7. The process as claimed in one or more of claims 3 to 6, wherein the silver (I) ions formed in the complex in stage (c) are present in the form of silver chloride.

8. The process as claimed in one or more of claims 3 to 7, wherein the skeleton silicate is treated in stage (a) with a solution containing $Ag^+$ ions in a concentration of 0.001 to 10 mol/liter, preferably 0.01 to 1 mol/liter, at room temperature, with the exclusion of light.

9. The process as claimed in one or more of claims 3 to 8, wherein the reduction in stage (b) is carried out at 100 to 500°C, preferably at 300 to 400°C, under a partial pressure of the reducing agent of 0.05 to 5.0 bar, preferably 0.4 to 0.75 bar.

10. The process as claimed in one or more of claims 3 to 9, wherein the re-oxidation in stage (c) is carried out at 100 to 350°C, preferably 250 to 290°C, under a partial pressure of the oxidizing agent of 0.05 to 5.0 bar, preferably 0.4 to 0.75 bar, in the course of 5 to 60, preferably 10 to 30, minutes.

11. The process as claimed in claim 10, wherein the re-oxidation is carried out using a halogen as the oxidizing agent, preferably at 100-200°C.

12. The process as claimed in one or more of claims 3 to 11, wherein the solution containing halide ions in stage (d) has a molar concentration of 0.001 to 2, preferably 0.01 to 0.5.

**Claims for the following Contracting State: ES**

1. A process for the preparation of a complex silicate catalyst of the formula

$$M^{n+}_{2/n}O \cdot v\,Ag^{o} \cdot w\,AgX \cdot Al_2O_3 \cdot x\,SiO_2 \cdot y\,H_2O \quad (1)$$

in which M denotes a metal atom of valence n, $Ag^{o}$ denotes elementary silver, X denotes a halogen atom and v, w, x and y denote numbers in the range from 0.1 to 7.5, which comprises (a) reacting a skeleton silicate with a silver salt, (b) converting the skeleton silicate containing silver ions by means of reducing agents into a silicate complex containing elementary silver, (c) converting this complex by partial re-oxidation with oxidizing agents into a silicate complex containing silver(I) ions and elementary silver and (d) then converting the complex into a silicate complex of the formula (1) by treatment with a solution containing halide ions in an alkaline medium.

2. The process as claimed in claim 1, wherein the catalyst corresponds to the formula

$$Na_2O \cdot 1.4\,Ag \cdot 0.4\,AgCl \cdot Al_2O_3 \cdot 2.47\,SiO_2 \cdot 3.9\,H_2O \quad (2).$$

3. The process as claimed in claim 1 or 2, wherein the reducing agent used is hydrogen.

4. The process as claimed in one or more of claims 1 to 3, wherein the oxidizing agent employed is oxygen or halogen, preferably chlorine, and water or hydrogen halide is liberated in the course of the re-oxidation.

5. The process as claimed in one or more of claims 1 to 4, wherein, when the re-oxidation is carried out with a halogen in stage (c), the subsequent treatment (d) is only carried out with an alkaline solution of a complex-former in the presence of a salt containing the metal ion M of formula (1).

6. The process as claimed in one or more of claims 1 to 5, wherein the silver(I) ions formed in the complex in stage (c) are present in the form of silver chloride.

7. The process as claimed in one or more of claims 1 to 6, wherein the skeleton silicate is treated in stage (a) with a solution containing $Ag^+$ ions in a concentration of 0.001 to 10 mol/liter, preferably 0.01 to 1 mol/liter, at room temperature, with the exclusion of light.

8. The process as claimed in one or more of claims 1 to 7, wherein the reduction in stage (b) is carried out at 100 to 500°C, preferably at 300 to 400°C, under a partial pressure of the reducing agent of 0.05 to 5.0 bar, preferably 0.4 to 0.75 bar.

9. The process as claimed in one or more of claims 1 to 8, wherein the re-oxidation in stage (c) is carried out at 100 to 350°C, preferably at 250 to 290°C, under a partial pressure of the oxidizing agent of 0.05 to 5.0 bar, preferably 0.4 to 0.75 bar, in the course of 5 to 60 minutes, preferably 10 to 30 minutes.

10. The process as claimed in claim 9, wherein the re- oxidation is carried out using halogen as the oxidizing agent, preferably at 100-200°C.

11. The process as claimed in one or more of claims 1 to 10, wherein the solution containing halide ions

in stage (d) has a molar concentration of 0.001 to 2, preferably 0.01 to 0.5.

## Revendications

### Revendications pour les Etats contractants suivants: BE, DE, IT, NL, FR, GB

1. Silicate complexe catalyseur dont la formule est :

$$M^{n+}_{2/n}O \cdot v\,Ag^{O} \cdot w\,AgX \cdot Al_2O_3 \cdot x\,SiO_2 \cdot y\,H_2O \quad (1)$$

où M est un atome de métal de valence n, $Ag^{O}$ l'argent élémentaire, X un atome d'halogène et v, w, x et y des nombres compris entre 0,1 et 7,5.

2. Catalyseur selon la revendication 1 caractérisé par la formule suivante :

$$Na_2O \cdot 1{,}4\,Ag \cdot 0{,}4\,AgCl \cdot Al_2O_3 \cdot 2{,}47\,SiO_2 \cdot 3{,}9\,H_2O \quad (2).$$

3. Procédé de fabrication d'un silicate complexe catalyseur, caractérisé en ce que (a) on fait réagir un silicate à squelette avec un sel d'argent, b) on transforme le silicate à squelette contenant des ions argent, au moyen de réducteurs, en un silicate complexe contenant de l'argent élémentaire, (c) on transforme ce complexe, par réoxydation partielle au moyen d'oxydants, en un silicate complexe contenant des ions argent(I) et de l'argent élémentaire, et (d) on transforme ensuite celui-ci, en le traitant par une solution contenant des ions halogénures en milieu alcalin, en un silicate complexe selon la revendication 1.

4. Procédé selon la revendication 3 caractérisé en ce que le réducteur utilisé est l'hydrogène.

5. Procédé selon la revendication 3 ou 4, caractérisé en ce que l'oxydant utilisé est l'oxygène ou un halogène, de préférence le chlore, et la réoxydation libère de l'eau ou un halogénure d'hydrogène.

6. Procédé selon l'une quelconque des revendications 3 à 5, caractérisé en ce que, lorsque la réoxydation a été effectuée par un halogène dans l'étape (c), le traitement ultérieur (d) est effectué seulement avec une solution alcaline d'un générateur de complexes en présence d'un sel contenant l'ion métallique M présent dans la formule (1).

7. Procédé selon l'une quelconque des revendications 3 à 6, caractérisé en ce que les ions argent (I) formés dans le complexe lors de l'étape (c) sont sous forme de chlorure d'argent.

8. Procédé selon l'une quelconque des revendications 3 à 7, caractérisé en ce que le silicate à squelette est traité dans l'étape (a) par une solution contenant des ions $Ag^+$ à une concentration comprise entre 0,001 et 10 mol/litre, de préférence entre 0,01 et 1 mol/litre, à la température ambiante et à l'abri de la lumière.

9. Procédé selon l'une quelconque des revendications 3 à 8, caractérisé en ce que la réduction de l'étape (b) est effectuée entre 100 et 550°C, de préférence entre 300 et 400°C, sous une pression partielle du réducteur comprise entre 0,05 et 5,0 bar, de préférence entre 0,4 et 0,75 bar.

10. Procédé selon l'une quelconque des revendications 3 à 9, caractérisé en ce que la réoxydation de l'étape (c) est effectuée entre 100 et 350°C, de préférence entre 250 et 290°C, sous une pression partielle de l'oxydant comprise entre 0,05 et 5,0 bar, de préférence entre 0,4 à 0,75 bar, pendant une durée de 5 à 60 minutes, de préférence de 10 à 30 min.

11. Procédé selon la revendication 10, caractérisé en ce que la réoxydation est effectuée avec un halogène comme oxydant de préférence à une température de 100 à 200°C .

12. Procédé selon l'une quelconque des revendications 3 à 11, caractérisé en ce que la solution contenant des ions halogénures dans l'étape (d) a une concentration molaire comprise entre 0,001 et 2, de préférence entre 0,01 et 0,5.

### Revendications pour l'Etat contractant suivant: ES

1. procédé de préparation d'un silicate complexe catalyseur dont la formule est :

$$M^{n+}_{2/n}O \cdot v\,Ag^{O} \cdot w\,AgX \cdot Al_2O_3 \cdot x\,SiO_2 \cdot y\,H_2O \quad (1)$$

où M est un atome de métal de valence n, $Ag^{O}$ l'argent élémentaire, X un atome d'halogène et v, w, x et y des nombres compris entre 0,1 et 7,5, procédé caractérisé en ce que (a) on fait réagir un silicate à squelette avec un sel d'argent, (b) on transforme le silicate à squelette contenant des ions argent, au moyen de réducteurs, en un silicate complexe contenant de l'argent élémentaire, (c) on transforme ce -complexe, par réoxydation partielle au moyen d'oxydants, en un silicate complexe contenant des ions argent(I) et de l'argent élémentaire, et (d) on transforme ensuite celui-ci, en le traitant par une solution contenant des ions halogénures en milieu alcalin, en un silicate complexe de formule (1).

2. Procédé selon la revendication 1 caractérisé en ce que le catalyseur répond à la formule suivante :

$$Na_2O \cdot 1{,}4\,Ag \cdot 0{,}4\,AgCl \cdot Al_2O_3 \cdot 2{,}47\,SiO_2 \cdot 3{,}9\,H_2O \quad (2).$$

3. Procédé selon la revendication 1 ou 2 caractérisé en ce qu'on utilise l'hydrogène comme réducteur.

4. Procédé selon l'une quelconque des revendications 1 à 3 caractérisé en ce qu'on utilise l'oxygène ou un halogène comme oxydant, de préférence le chlore, et en ce que la réoxydation libère de l'eau ou un halogénure d'hydrogène.

5. Procédé selon l'une quelconque des revendications 1 à 4 caractérisé en ce que lorsque la réoxydation a été effectuée par un halogène dans l'étape (c), le traitement ultérieur (d) est effectué seulement avec une solution alcaline d'un générateur de complexes en présence d'un sel contenant l'ion métallique M présent dans la formule (1).

6. Procédé selon l'une quelconque des revendications 1 à 5 caractérisé en ce que les ions argent (I) formés dans le complexe lors de l'étape (c) sont sous forme de chlorure d'argent.

7. Procédé selon l'une quelconque des revendications 1 à 6 caractérisé en ce que le silicate à squelette est traité dans l'étape (a) par une solution contenant des ions $Ag^+$ à une concentration comprise entre 0,001 et 10 mol/litre, de préférence entre 0,01 et 1 mol/litre, à la température ambiante et à l'abri de la lumière.

8. Procédé selon l'une quelconque des revendications 1 à 7 caractérisé en ce que la réduction de l'étape (b) est effectuée entre 100 et 550°C, de préférence entre 300 et 400°C, sous une pression partielle du réducteur comprise entre 0,05 et 5,0 bar, de préférence entre 0,4 et 0,75 bar.

9. Procédé selon l'une quelconque des revendications 1 à 8 caractérisé en ce que la réoxydation de l'étape (c) est effectuée entre 100 et 350°C, de préférence entre 250 et 290°C, sous une pression partielle de l'oxydant comprise entre 0,05 et 5,0 bar, de préférence entre 0,4 à 0,75 bar, pendant une durée de 5 à 60 minutes, de préférence de 10 à 30 min.

10. Procédé selon la revendication 9 caractérisé en ce que la réoxydation est effectuée avec un halogène comme oxydant, de préférence à une température de 100 à 200°C.

11. Procédé selon l'une quelconque des revendications 1 à 10 caractérisé en ce que la solution contenant des ions halogénures dans l'étape (d) a une concentration molaire comprise entre 0,001 et 2, de préférence entre 0,01 et 0,5.